# EUROPEAN PATENT APPLICATION

(11) **EP 3 875 465 A1**
(43) Date of publication of application: **08.09.2021**
(21) Application number: 19878441.5
(22) Date of filing: 21.06.2019
(51) Int. Cl.: C07K 1/107, C07K 14/605, A61K 38/26, A61K 47/60, A61P 25/28, A61P 3/04, A61P 3/10

(54) **METHOD FOR MODIFICATION OF POLYPETIDE AND USES**

(30) Priority: 31.10.2018 CN 201811283889
(71) Applicant: Xiangya Hospital, Central South University, Changsha, Hunan 410008 (CN)
(72) Inventor: CHEN, Yongheng, Changsha, Hunan 410008 (CN); ZHOU, Zhan, Changsha, Hunan 410008 (CN); CHEN, Zhuchu, Changsha, Hunan 410008 (CN); LI, Jun, Changsha, Hunan 410008 (CN)
(74) Representative: Cesa, Roberta
(86) International application number: PCT/CN2019/092232
(87) International publication number: WO 2020/087947

(57) **Abstract**

Provided are a method for the modification of a polypeptide and uses. The method comprises the following steps: (1) introducing an X into the N-terminus of a polypeptide, thereby obtaining X-polypeptide; (2) oxidizing the X into an aldehyde group; (3) adding a reducing agent, and covalently coupling the oxidation product obtained in step (2) with PEG, thereby obtaining a PEG-modified polypeptide, wherein X is threonine or serine. In the present application, a single component of PEG-modified polypeptide is obtained by introducing a threonine or serine into the N-terminus of the polypeptide, and deriving the amino alcohol structure at the ortho-position of the N-terminus of the polypeptide as an aldehyde group by using a high-specificity oxidation method and covalently coupling the aldehyde group with PEG. The method has a strong universality and a wide range of application, and the method for separating the modified polypeptide is simple and convenient, thereby improving the stability and the circulating half-life of the polypeptide.

SEQUENCE LISTING

<110>  XIANGYA HOSPITAL, CENTRAL SOUTH UNIVERSITY

<120>  METHOD FOR MODIFICATION OF POLYPETIDE AND USES

<130>  PCT190554PPC

<160>  2

<170>  PatentIn version 3.3

<210>  1
<211>  31
<212>  PRT
<213>  Artificial sequence

<220>
<223>  GLP-1(7-37)

<400>  1

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  2
<211>  30
<212>  PRT
<213>  Artificial sequence

<220>
<223>  GLP-1(7-36)

<400>  2

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

## Description

### TECHNICAL FIELD

The present application belongs to the field of medical technology, and relates to a method for the modification of a polypeptide and uses and, in particular, to a GLP-1 receptor agonist analog, a preparation method therefor and use thereof.

### BACKGROUND

Glucagon-like peptide-1 (GLP-1), a glucose-dependent hypoglycemic polypeptide hormone secreted by intestinal L cells, has effects of preventing pancreatic cell degeneration, stimulating pancreatic cell proliferation and differentiation, and promoting insulin production, and is an intestinal peptide hormone with the most potent insulin secretion function that has been found. GLP-1 can improve the blood glucose of patients with type II diabetes by a variety of mechanisms such as promoting the regeneration and repair of islet β cells and increasing the number of islet β cells. In addition, GLP-1 can slow gastric emptying rate, inhibit appetite by acting on the hypothalamus and reduce food intake to control blood glucose. GLP-1 has a good application prospect in the treatment of type II diabetes and has become a research hotspot in the field of diabetes treatment in recent years.

GLP-1 consists of 30 amino acids and includes two biologically active forms: GLP-1(7-37) amide and GLP-1(7-36) amide, which differ by only one amino acid sequence. Approximately 80% of the circulating activity of GLP-1 is derived from GLP-1(7-36) amide. The molecular weight of GLP-1 is small, only 3 kDa, and thus GLP-1 can be easily filtered and removed by glomerulus; meanwhile, since the His-Ala sequence at the N-terminus of GLP-1 is a recognition site of dipeptidyl peptidase IV, GLP-1 can be easily degraded by dipeptidyl peptidase IV *in vivo,* and the activity of residual fragments after enzymatic hydrolysis is only one percent of the activity of the original GLP-1. Therefore, the half-life of GLP-1 is very short (about 2 minutes) *in vivo,* the bioavailability of the drug is low, and thus the drug needs to be frequently injected in clinical use to maintain the plasma concentration, which brings additional pain to the patient and greatly limits the clinical application of GLP-1. Improving the stability of GLP-1 *in vivo* and prolonging the drug half-life are urgent problems to be solved.

Currently, the modification of GLP-1 mainly includes two aspects: in patents CN1329620A and CN1350548A, the amino acid sequence of GLP-1 is modified to achieve the effects of reducing the enzymatic hydrolysis rate and improving the circulating stability; in patent CN101337989A, fatty acid chains are coupled to the amino acid sequence of GLP-1 to increase the molecular weight of GLP-1, thereby improving the affinity of GLP-1 to plasma and prolonging the half-life; the marketed drug Liraglutide is obtained by replacing lysine at position 34 of GLP-1 with arginine and coupling a C-16 fatty acid and a glutamic acid spacer at position 26 of lysine, prolonging the half-life of the drug to 13 hours, but the drug compliance of Liraglutide still needs to be improved, its chain-coupled fatty acid solubility is not high, its water solubility is poor, and its N-terminus His-Ala sequence is still recognized and degraded by dipeptidase, resulting in the reduction of half-life.

The polyethyleneglycol (PEG) modification of proteins and polypeptides is a mature modification method, which can improve drug stability, improve drug solubility, reduce drug immunogenicity, increase resistance to enzymatic hydrolysis, prolong the drug half-life, and improve pharmacokinetic properties *in vivo.* So far, 12 PEG-modified drugs have been approved by FDA and marketed, and more than 40 PEG-modified drugs are in different clinical experimental stages. PEG modification of GLP-1 is an effective way to solve the clinical application problems of GLP-1.

Kang Choon Lee et al. performed PEG modification on the N-terminus and intrachain amino groups of GLP-1 to obtain an active single modification product to a certain extent. However, due to the limitation of the amino modification method itself, there are problems such as uncertainty of the modification site and difficulty in separation, and thus a uniform single modification product cannot be obtained.

In CN107266555A and CN107266557A, a certain amino acid in the GLP-1 sequence is mutated to cysteine by site-directed mutagenesis and is subjected to site-directed modification with PEG maleimide to prolong the half-life of the drug to a certain extent. However, since the sequence of the amino acid is not exactly the same as the original GLP-1, the homology decreases, which may enhance the immunogenicity of the drug *in vivo* and reduce the pharmacodynamics *in vivo.* In CN1372570A and CN101125207A, the GLP-1 analog Exendin-4 is subjected to PEG modification, but there are also problems of immunogenicity and drug resistance.

CN106421471A discloses a new type of Xenopus laevis glucagon-like peptide-1 (GLP-1) conjugated peptide, as well as application thereof. A spiral promoting sequence is introduced to N-terminus of the Xenopus laevis GLP-1, and meanwhile, PEG-based modification is performed to obtain an analog, with reserved hypoglycemic activity and longer pharmacological action time, of the Xenopus laevis GLP-1. However, the method also requires cysteine modification of GLP-1, which may enhance the immunogenicity of the drug *in vivo.*

Therefore, it is important in the field of medicine technology to provide a polypeptide modification method for PEG modification of GLP-1 receptor agonists, which is good in universality and simple in process, to improve component uniformity, drug stability, and circulating half-life of GLP-1 receptor agonists.

### SUMMARY

In view of the deficiencies of the existing art, the present application provides a method for the modification of a polypeptide and uses. In the method, threonine or serine is introduced to the N-terminus of the polypeptide, thereby achieving the site-directed PEG modification of the polypeptide and improving composition uniformity, stability, and circulating half-life of the polypeptide.

To achieve this object, the present application adopts technical solutions described below.

In a first aspect, the present application provides a method for modification of a polypeptide, comprising the following steps:
(1) introducing an X to the N-terminus of a polypeptide to obtain an X-polypeptide;
(2) oxidizing the X into an aldehyde group, for example, the reaction is as shown by Formula (A): 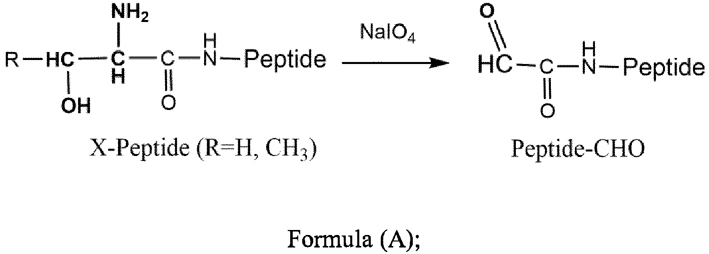 and
(3) adding a reducing agent, and covalently coupling the oxidation product obtained in step (2) with PEG to obtain a PEG-modified polypeptide, for example, the reaction is as shown by Formula (B): 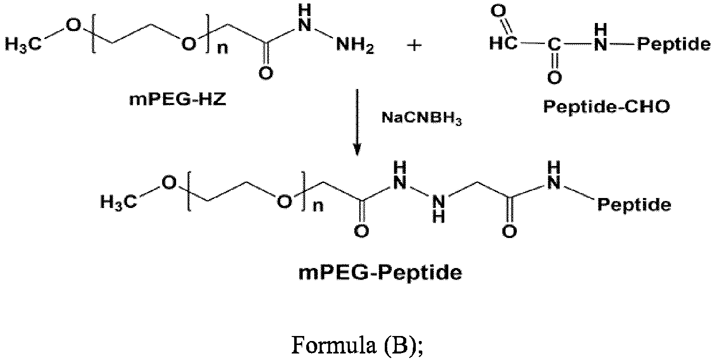
wherein X is threonine or serine.

In the present application, a threonine or serine is introduced in a site-directed manner to the N-terminus of the polypeptide, the amino alcohol structure at the ortho-position of the N-terminus of the polypeptide is derived as an aldehyde group by using a high specific oxidation method, and the aldehyde group is covalently coupled with PEG capable of being reacted with the aldehyde group at the end group, achieving the purpose of site-directed modification of the polypeptide; meanwhile, the threonine or serine introduced at the N-terminus has been removed during the oxidation reaction, leaving only one -CH₂-CO-structure at the N-terminus of the original polypeptide, effectively retaining the biological activity of the original polypeptide without altering the sequence of the original polypeptide.

Preferably, the method of the introducing in step (1) includes a solid-phase synthesis method or a biological expression method.

Preferably, the solid-phase synthesis method is Fmoc method.

Preferably, the biological expression method includes transforming a constructed X-polypeptide expression vector into host bacteria, inducing and collecting the bacteria, and performing lysing and purification to obtain the X-polypeptide.

Preferably, the oxidizing in step (2) is carried out with an oxidizing agent.

The oxidizing agent in the present application has an oxidation effect only on the amino alcohol structure at the ortho-position, and thus has a highly specific oxidation effect on the N-terminal threonine or serine of the polypeptide, thereby achieving the effect of oxidizing the N-terminal threonine or serine to the aldehyde group.

Preferably, the oxidizing agent includes a periodate oxidizing agent, preferably sodium periodate.

Preferably, the molar ratio of the oxidizing agent to the X-polypeptide is (1-3):1, for example, 1:1, 1:2 or 1:3.

Preferably, the oxidizing in step (2) is carried out at a temperature of 3 °C to 6 °C, for example, 3 °C, 4 °C, 5 °C or 6 °C, preferably 3 °C to 4 °C.

Preferably, the oxidizing in step (2) is carried out for 20 min to 40 min, for example, 20 min, 21 min, 22 min, 23 min, 24 min, 25 min, 26 min, 27 min, 28 min, 29 min, 30 min, 31 min, 32 min, 33 min, 34 min, 35 min, 36 min, 37 min, 38 min, 39 min or 40 min, preferably 30 min to 35 min.

Preferably, the reducing agent in step (3) includes any one or a combination of at least two of sodium borohydride, sodium borohydride acetate or sodium cyanoborohydride, preferably sodium cyanoborohydride.

The reducing agent in the present application is used for reducing double bonds generated during the coupling reaction, facilitating the progress of the coupling reaction and maintaining the stability of the coupling product.

Preferably, the PEG in step (3) is methoxypolyethylene glycol.

Preferably, an end group of methoxypolyethylene glycol in step (3) includes any one of an amino group, an oxyamino group or hydrazide.

Preferably, the molar ratio of the PEG to the oxidation product in step (3) is (4-6):1, for example, 4:1, 5:1 or 6:1.

Preferably, the covalently coupling in step (3) is carried out at a temperature of 3 °C to 6 °C, for example, 3 °C, 4 °C, 5 °C or 6 °C, preferably 3 °C to 4 °C.

Preferably, the covalently coupling in step (3) is carried out for 1 hour (h) to 3 h, for example, 1 h, 2 h or 3 h.

Preferably, the covalently coupling in step (3) is carried out at a pH of 4 to 5, for example, 4, 4.1, 4.2, 4.3, 4.4, 4.5, 4.6, 4.7, 4.8, 4.9 or 5, preferably 4 to 4.5.

As a preferred solution, the present application provides a method for the modification of a polypeptide, comprising the following steps:
(1) introducing an X to the N-terminus of a polypeptide in a solid-phase synthesis method or a biological expression method to obtain an X-polypeptide;
(2) adding a periodate oxidizing agent at a molar ratio of the oxidizing agent to the X-polypeptide of (1-3): 1, and reacting for 20 minutes to 40 minutes at 3 °C to 6 °C, and to oxidize the X to an aldehyde group; and
(3) adding a reducing agent, and covalently coupling the oxidation product obtained in step (2) with methoxypolyethylene glycol at 3 °C to 6 °C at pH of 4 to 5 for 1 h to 3 h, wherein the molar ratio of the methoxypolyethylene glycol to the oxidation product is (4-6): 1, to obtain a PEG-modified polypeptide;
wherein X is threonine or serine.

In a second aspect, the present application provides a polypeptide analog, which is prepared by using the method described in the first aspect.

In a third aspect, the present application provides a GLP-1 receptor agonist analog, which is prepared by using the method described in the first aspect.

Preferably, the GLP-1 receptor agonist has a structure of PEG-X-GLP-1 receptor agonist;
wherein X is threonine or serine.

In the present application, by introducing threonine (Thr) or serine (Ser) to the N-terminus of a GLP-1 receptor agonist, not only the site-directed mono-modification of PEG on a GLP-1 receptor agonist is achieved to obtain a GLP-1 receptor agonist analog having a uniform composition, but also the N-terminal His-Ala sequence of GLP-1 is effectively prevented from being degraded by dipeptidyl peptidase IV, thereby improving the stability of the GLP-1 receptor agonist and prolonging the half-life.

Preferably, the GLP-1 receptor agonist includes any one of GLP-1, exenatide, liraglutide, albiglutide, dulaglutide, lixisenatide, benaglutide or semaglutide.

In the present application, GLP-1 includes GLP-1(7-37) amide or GLP-1(7-36) amide, wherein the amino acid sequence of GLP-1(7-37) is as shown in SEQ ID NO.1:
SEQ ID NO.1: HAEGTFTSDVSSYLEGQAAKEFIAWLVKGRG;

The amino acid sequence of GLP-1(7-36) is as shown in SEQ ID NO.2:
SEQ ID NO.2: HAEGTFTSDVSSYLEGQAAKEFIAWLVKGR-NH₂.

Preferably, the PEG is methoxypolyethylene glycol (mPEG).

In the present application, the PEG may be a linear PEG or a branched PEG, preferably a linear PEG.

Preferably, the end group of methoxypolyethylene glycol includes any one of an amino group, an oxyamino group or hydrazide to obtain any one of mPEG amine (mPEG-NH₂), mPEG oxyamine (mPEG-O-NF₂) or an mPEG hydrazide (mPEG-CO-NH-NH₂).

In the present application, the site-directed covalent coupling of PEG on a GLP-1 receptor agonist is achieved by reacting PEG whose end group is an amino group, an oxyamino group or hydrazide with an aldehyde group in the presence of a reducing agent.

Preferably, the molecular weight of methoxypolyethylene glycol is 2000 Da to 50000 Da, for example, 2000 Da, 5000 Da, 10000 Da, 15000 Da, 20000 Da, 25000 Da, 30000 Da, 35000 Da, 40000 Da, 45000 Da or 50000 Da, preferably 5000 Da to 20000 Da, further preferably 5000 Da to 10000 Da.

In a fourth aspect, the present application provides a pharmaceutical composition, comprising the polypeptide analog described in the second aspect and/or the GLP-1 receptor agonist analog described in the third aspect.

Preferably, the pharmaceutical composition further includes any one or a combination of at least two of a pharmaceutically acceptable carrier, excipient or diluent.

In a fifth aspect, the present application provides use of any one or a combination of at least two of the polypeptide analog described in the second aspect, the GLP-1 receptor agonist analog described in the third aspect or the pharmaceutical composition described in the fourth aspect in the preparation of a medicament for the prevention and/or treatment of obesity, diabetes or Alzheimer's disease.

Compared with the existing art, the present application has beneficial effects described below.
(1) In the present disclosure, a PEG-modified polypeptide with a single component is obtained by introducing a threonine or serine to the N-terminus of the polypeptide by using a solid-phase synthesis method or a biological expression method, deriving the amino alcohol structure at the ortho-position of the N-terminus of the polypeptide as an aldehyde group by using a high-specificity oxidation method, and covalently coupling the aldehyde group with PEG. The method has a strong universality and a wide range of application, and the method for separating the modified polypeptide is simple and convenient, thereby improving the stability and the circulating half-life of the polypeptide.
(2) The GLP-1 receptor agonist analog prepared by the method for modification of a polypeptide of the present application has 100% homology with the GLP-1 receptor agonist, thereby preserving the hypoglycemic effect of the GLP-1 receptor agonist and avoiding problems of immunogenicity and drug resistance.
(3) The overall modification rate of the PEG-modified GLP-1 receptor agonist of the present application is 80.3%.
(4) In the present application, a threonine or a serine is introduced to the N-terminus of GLP-1, which effectively prevents the N-terminal His-Ala sequence of GLP-1 from being degraded by dipeptidyl peptidase IV, and the in vivo half-life of the obtained GLP-1 analog is increased by more than 60-fold and the AUC value is 10-fold higher than that of the original GLP-1.

### BRIEF DESCRIPTION OF DRAWINGS

FIG. 1 is an SDS-PAGE electropherogram of T-GLP-1, where 1 represents standard protein samples whose molecular weights are 14.4 KDa, 18.4 KDa, 25.0 KDa, 35.0 KDa, 45.0 KDa, 66.2 KDa, and 116.0 KDa in sequence, 2 represents a T-GLP-1-containing mixture of E. coli soluble expression, 3 represents T-GLP-1 initially purified by affinity chromatography, and 4 represents T-GLP-1 obtained by ion-exchange chromatography; and
FIG. 2 is a diagram of high performance liquid chromatography (HPLC) of T-GLP-1, T-GLP-1, and mPEG₅ₖ-T-GLP-1.

### DETAILED DESCRIPTION

To further elaborate the technical means adopted and the effects achieved in the present application, the present application is described below in conjunction with the examples and drawings. It is to be understood that the specific examples set forth below are intended to illustrate but not to limit the present application.

Experiments without specific techniques or conditions noted in the examples are conducted according to techniques or conditions described in the literature in the art or a product specification. The reagents or instruments used herein without manufacturers specified are conventional products commercially available from proper channels.

### Example 1 Synthesis of X-GLP-1 receptor agonist by Fmoc solid-phase method

The activated Fmoc-rink amide-MBHA resin was placed in a CS Bio polypeptide synthesizer and was connected to amino acids according to the sequence of the X-GLP-1 receptor agonist through the deprotection and coupling steps sequentially to obtain a resin to which the X-GLP-1 receptor agonist was connected. The efficiency of the coupling step was measured by the ninhydrin method, and if the color reaction was negative, go to the next coupling cycle. After the completion of the reaction, the polypeptide was lysed from the resin by adding a lysis buffer, and a crude X-GLP-1 receptor agonist was obtained after washing. The crude product was purified by preparative HPLC, the mobile phase was A (water + 0.1% TFA) and B (acetonitrile + 0.1% TFA), the target peak was collected, and the crude product was freeze-dried to obtain the pure X-GLP-1 receptor agonist.

In this example, X is threonine or serine, and the GLP-1 receptor agonist is GLP-1, exenatide, liraglutide, albiglutide, dulaglutide, lixisenatide, benalglutide or semaglutide.

### Example 2 Biological expression of X-GLP-1 receptor agonist

A threonine codon or a serine codon was added to the end of a GLP-1 receptor agonist gene fragment and constructed to the upstream of the enterokinase site (DDDDK) and the histidine tag in the pET28a vector to obtain an expression plasmid of the fused peptide Histag-DDDK-T-GLP-1. The constructed plasmid was transformed into Escherichia coli BL21 (Takara) and induced with 0.5 mM IPTG, and the bacteria were collected. After the bacteria were lysed, the supernatant was collected by centrifugation and initially purified using a Ni affinity column, then the enterokinase site sequence and histidine tag were removed by enterokinase digestion, and finally, the supernatant was purified using ion-exchange chromatography to obtain the X-GLP-1 receptor agonist.

In this example, X is threonine or serine, and the GLP-1 receptor agonist is GLP-1, exenatide, liraglutide, albiglutide, dulaglutide, lixisenatide, benalglutide or semaglutide.

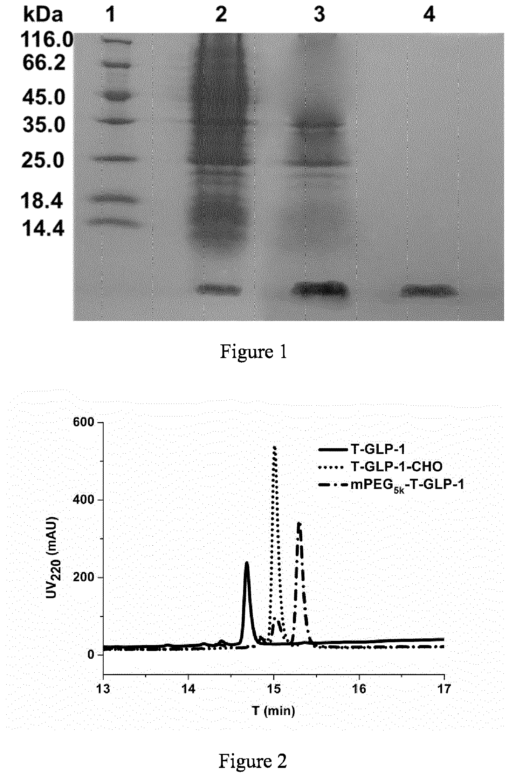

The sample during the expression and purification process was collected for SDS-PAGE detection. As shown in FIG. 1, for example, the purity of the obtained T-GLP-1 was more than 95%.

### Example 3 N-terminal site-directed oxidation of X-GLP-1 receptor agonists

The X-GLP-1 receptor agonist was dissolved in the PB buffer (50 mM, pH = 7.0), 1 mg/mL NaIO₄ solution was added at the molar ratio of NaIO₄ to the X-GLP-1 receptor agonist of 2:1, the reaction was carried out at 4 °C for 30 min, and 100 µL of ethylene glycol was added to terminate the reaction.

The sample was added to a GE G25 desalination column, the polypeptide peak was collected, and an achromatic magenta color reagent was added for color development.

It was found from color development results that the oxidized X-GLP-1 receptor agonist was bright red, a feature color of the aldehyde group in the achromatic magenta detection, indicating that NaIO4 oxidized the threonine or serine to the aldehyde group.

In this example, X is threonine or serine, and the GLP-1 receptor agonist is GLP-1, exenatide, liraglutide, albiglutide, dulaglutide, lixisenatide, benalglutide or semaglutide.

The samples before and after oxidation were subjected to high performance liquid chromatography. The detection results were shown in FIG. 2. For example, the retention time of T-GLP-1 on the C18 column was 14.68 min, and the retention time of T-GLP-1-CHO formed after oxidation with the unique aldehyde group was 15.01 min.

### Example 4 Preparation of mPEG₅ₖ-HZ-modified GLP-1 analog

The mPEG hydrazide powder (mPEG₅ₖ-HZ) with a molecular weight of 5000 Da was added to the oxidized Thr-GLP-1, where the molar ratio of mPEG₅ₖ-HZ to Thr-GLP-1 was 5:1. 5 mM of sodium cyanoborohydride was added as a reducing agent during the reaction, and the oscillatory reaction was carried out at pH of 4.5 for 2 h at 4 °C.

After the completion of the reaction, the reaction solution was subjected to chromatographic separation on the GE Superdex 75 10/300 GL column, and the mobile phase was a Na₂SO₄ buffer system (0.1 M, pH = 7.4) containing 20 mM of PB at a flow rate of 0.6 mL/min. The peak was collected at a detection wavelength of 220 nm. The collected sample was dialyzed overnight in a system containing 20 mM of PB and 5% mannitol and then stored after ultrafiltration and concentration.

Results are shown in FIG. 2, and the retention time of the successfully separated and purified mPEG₅ₖ-T-GLP-1 reached 15.3 min, with an overall modification rate of 80.3%.

### Example 5 Preparation of mPEG₂₀ₖ-HZ-modified exenatide analog

The mPEG hydrazide powder (mPEG₂₀ₖ-HZ) with a molecular weight of 20000 Da was added to the oxidized Thr-exenatide, where the molar ratio of mPEG₂₀ₖ-HZ to Thr-exenatide was 5:1. 5 mM of sodium cyanoborohydride was added as a reducing agent during the reaction, and the oscillatory reaction was carried out at pH of 4.5 for 2 h at 4 °C.

After the completion of the reaction, the reaction solution was subjected to chromatographic separation on the GE Superdex 75 10/300 GL column, and the mobile phase was a Na₂SO₄ buffer system (0.1 M, pH = 7.4) containing 20 mM of PB at a flow rate of 0.6 mL/min. The peak was collected at a detection wavelength of 220 nm. The collected sample was dialyzed overnight in a system containing 20 mM of PB and 5% mannitol and then stored after ultrafiltration and concentration.

It was found from the results that mPEG₂₀k-HZ-modified exenatide was successfully separated and purified.

### Example 6 Preparation of mPEG₁₀ₖ-O-NH₂-modified liraglutide analog

The mPEG oxyammonia powder (mPEG₁₀ₖ-O-NH₂) with a molecular weight of 10000 Da was added to the oxidized Thr-liraglutide, where the molar ratio of mPEG₁₀ₖ-O-NH₂ to Thr-liraglutide was 5:1. 5 mM of sodium cyanoborohydride was added as a reducing agent during the reaction, and the oscillatory reaction was carried out at pH of 4.5 for 2 h at 4 °C.

After the completion of the reaction, the reaction solution was subjected to chromatographic separation on the GE Superdex 75 10/300 GL column, and the collected sample was detected by HPLC. It was found that the mPEG₁₀ₖ-O-NH₂-modified liraglutide was successfully separated and purified.

### Example 7 Preparation of mPEG₂ₖ-NH₂-modified albiglutide analog

The mPEG amino powder (mPEG₂ₖ-NH₂) with a molecular weight of 2000 Da was added to the oxidized Ser-albiglutide, where the molar ratio of mPEG₂ₖ-NH₂ to Ser-albiglutide was 6:1. 5 mM of sodium borohydride was added as a reducing agent during the reaction, and the oscillatory reaction was carried out at pH of 4 for 3 h at 3 °C.

After the completion of the reaction, the reaction solution was subjected to chromatographic separation on the GE Superdex 75 10/300 GL column, and the collected sample was detected by HPLC. It was found that the mPEG₂ₖ-NH₂-modified albiglutide was successfully separated and purified.

### Example 8 Preparation of mPEG₅₀ₖ-NH₂-modified dulaglutide analog

The mPEG amino powder (mPEG₅₀ₖ-NH₂) with a molecular weight of 50000 Da was added to the oxidized Ser-dulaglutide, where the molar ratio of mPEG₅₀ₖ-NH₂ to Ser-dulaglutide was 4:1. 5 mM of sodium borohydride acetate was added as a reducing agent during the reaction, and the oscillatory reaction was carried out at pH of 5 for 1 h at 6 °C.

After the completion of the reaction, the reaction solution was subjected to chromatographic separation on the GE Superdex 75 10/300 GL column, and the collected sample was detected by HPLC. It was found that the mPEG₅₀ₖ-NH₂-modified dulaglutide was successfully separated and purified.

### Example 9 Preparation of mPEG₅ₖ-HZ-modified semaglutide analog

The mPEG hydrazide powder (mPEG₅ₖ-HZ) with a molecular weight of 5000 Da was added to the oxidized Thr-semaglutide, where the molar ratio of mPEG₅ₖ-HZ to Thr-semaglutide was 5:1. 5 mM of sodium cyanoborohydride was added as a reducing agent during the reaction, and the oscillatory reaction was carried out at pH of 4.5 for 2 h at 4 °C.

After the completion of the reaction, the reaction solution was subjected to chromatographic separation on the GE Superdex 75 10/300 GL column, and the collected sample was detected by HPLC. It was found that the mPEG₅₀ₖ-NH₂-modified semaglutide was successfully separated and purified.

### Example 10 Preparation of mPEG₅ₖ-HZ-modified thymosin analog

The mPEG hydrazide powder (mPEG₅ₖ-HZ) with a molecular weight of 5000 Da was added to the oxidized Ser-thymosin, where the molar ratio of mPEG₅ₖ-HZ to Ser-thymosin was 5:1. 5 mM of sodium cyanoborohydride was added as a reducing agent during the reaction, and the oscillatory reaction was carried out at pH of 4.5 for 2 h at 4 °C.

After the completion of the reaction, the reaction solution was subjected to chromatographic separation on the GE Superdex 75 10/300 GL column, and the collected sample was detected by HPLC. It was found that the mPEG₅₀ₖ-NH₂-modified thymosin was successfully separated and purified.

### Example 11 In vivo pharmacokinetics detection of mPEG₅ₖ-T-GLP-1

The in vivo pharmacokinetic activity detection of mPEG₅ₖ-T-GLP-1 was carried out with 30 male SD rats, aged 7-8 weeks, weighing 200-250 g/rat. These rats were randomly grouped and injected with GLP-1 or mPEG₅ₖ-T-GLP-1 at a dose of 2 µg/kg by weight, and the injection method was subcutaneous injection.

Blood was taken from the eye socket of each rat at 1, 2, 4, 8, 30, 60, 120 and 240 min, timed from the first injection, then placed in tubes containing EDTA and centrifuged at 5000 rpm for 5 min at 4 °C, the cells of the lower layer were discarded, and the supernatant was stored at -80 °C.

The stored samples were all taken out and thawed. The GLP-1 concentration at different time points was measured using the rat GLP-1 enzyme-linked immunoassay ELISA testing kit, and the half-life and AUC value of GLP-1 before and after PEG modification were calculated based on the measurement results.

The stored samples were all taken out and thawed. The GLP-1 concentration at different time points was measured using the rat GLP-1 enzyme-linked immunoassay ELISA testing kit, and the half-life and AUC value of GLP-1 before and after PEG modification were calculated based on the measurement results.

The results show that the half-life of mPEG5k-T-GLP-1 *in vivo* is increased by more than 60-fold, and the AUC value is 10-fold higher than that of the original GLP-1.

### Example 12 In vivo pharmacodynamics activity assay of mPEG₅ₖ-T-GLP-1

The in vivo pharmacodynamics activity detection of mPEG₅ₖ-T-GLP-1 was carried out with 30 type II diabetic db/db mice, aged 7-8 weeks, weighing 8-250 g/mouse. These mice were fed a high-fat diet and made into models. These mice were observed daily from the start of animal feeding. Each of the mice was weighed every Wednesday after 8 hours of fasting, and meanwhile, the tail vein blood glucose levels of the mice were measured.

After 8 weeks of intervention, 18 mice were randomly selected and then divided into three groups. Each of these 18 mice was injected with GLP-1 or mPEG₅ₖ-T-GLP-1 at a dose of 2 µg/kg by weight, and the injection method was subcutaneous injection.

Blood was taken from the tail vein of each mouse at 1, 2, 4, 8, 30, 60, 120 and 240 min, timed from the first injection, then placed in tubes containing EDTA and centrifuged at 5000 rpm for 5 min at 4 °C, the cells of the lower layer were discarded, and the supernatant was stored at -80 °C.

The stored samples were all taken out and thawed, and the blood glucose concentration and insulin content were measured using the enzyme-linked immunoassay kit.

The results show that mPEG₅ₖ-T-GLP-1 had a significant hypoglycemic effect *in vivo,* and after 5 hours after injection, the blood glucose of db/db mice reached the normal level.

In summary, in the present disclosure, a PEG-modified polypeptide with a single component is obtained by introducing a threonine or serine into the N-terminus of the polypeptide by using a solid-phase synthesis method or a biological expression method, deriving the amino alcohol structure at the ortho-position of the N-terminus of the polypeptide as an aldehyde group by using a high-specificity oxidation method, and covalently coupling the aldehyde group with PEG. The method has a robust universality and a wide range of application, and the method for separating the modified polypeptide is simple and convenient, thereby improving the stability and the circulating half-life of the polypeptide. The GLP-1 receptor agonist analog prepared by the method for modification of a polypeptide of the present application has 100% homology with the GLP-1 receptor agonist, thereby preserving the hypoglycemic effect of the GLP-1 receptor agonist and avoiding problems of immunogenicity and drug resistance. The introduced threonine or serine effectively prevents the N-terminal His-Ala sequence of GLP-1 from being degraded by dipeptidyl peptidase IV, and the half-life of the obtained GLP-1 analog *in vivo* is increased by more than 60-fold, and the AUC value is 10-fold higher than that of the original GLP-1.

The applicant has stated that although the detailed method of the present application is described through the examples described above, the present application is not limited to the detailed method described above, which means that implementation of the present application does not necessarily depend on the detailed method described above. It should be apparent to those skilled in the art that any improvements made to the present application, equivalent replacements of raw materials of the product of the present application, additions of adjuvant ingredients to the product of the present application, and selections of specific manners, etc., all fall within the protection scope and the disclosed scope of the present application.

## Claims

1. A method for modification of a polypeptide, comprising the following steps:
(1) introducing an X to the N-terminus of a polypeptide to obtain an X-polypeptide;
(2) oxidizing the X into an aldehyde group; and
(3) adding a reducing agent, and covalently coupling the oxidation product obtained in step (2) with polyethyleneglycol (PEG) to obtain a PEG-modified polypeptide;
wherein X is threonine or serine.

2. The method according to claim 1, wherein the method of the introducing in step (1) comprises a solid-phase synthesis method or a biological expression method.

3. The method according to claim 2, wherein the solid-phase synthesis method is a Fmoc method.

4. The method according to claim 2, wherein the biological expression method comprises transforming a constructed X-polypeptide expression vector into host bacteria, inducing and collecting the bacteria, and performing lysing and purification to obtain the X-polypeptide.

5. The method according to any one of claims 1 to 4, wherein the oxidizing in step (2) is carried out with an oxidizing agent;
preferably, the oxidizing agent comprises a periodate, preferably sodium periodate;
preferably, the molar ratio of the oxidizing agent to the X-polypeptide is (1-3):1;
preferably, the oxidizing in step (2) is carried out at a temperature of 3 °C to 6 °C, preferably 3 °C to 4 °C;
preferably, the oxidizing in step (2) is carried out for 20 minutes to 40 minutes, preferably 30 minutes to 35 minutes.

6. The method according to any one of claims 1 to 5, wherein the reducing agent in step (3) comprises any one or a combination of at least two of sodium borohydride, sodium borohydride acetate or sodium cyanoborohydride, preferably sodium cyanoborohydride.

7. The method according to any one of claims 1 to 6, wherein the PEG in step (3) is methoxypolyethylene glycol;
preferably, an end group of methoxypolyethylene glycol in step (3) comprises any one of an amino group, an oxyamino group or hydrazide;
preferably, the molar ratio of the PEG to the oxidation product in step (3) is (4-6):1;
preferably, the covalently coupling in step (3) is carried out at a temperature of 3 °C to 6 °C, preferably 3 °C to 4 °C;
preferably, the covalently coupling in step (3) is carried out for 1 hour to 3 hours;
preferably, the covalently coupling in step (3) is carried out at a pH of 4 to 5, preferably 4 to 4.5.

8. The method according to any one of claims 1 to 7, comprising the following steps:
(1) introducing an X to the N-terminus of a polypeptide in a solid-phase synthesis method or a biological expression method to obtain an X-polypeptide;
(2) adding a periodate oxidizing agent at a molar ratio of the oxidizing agent to the X-polypeptide of (1-3): 1, and reacting for 20 minutes to 40 minutes at 3 °C to 6 °C, to oxidize the X to an aldehyde group; and
(3) adding a reducing agent, and covalently coupling the oxidation product obtained in step (2) with methoxypolyethylene glycol at 3 °C to 6 °C at pH of 4 to 5 for 1 hour to 3 hours, wherein the molar ratio of the methoxypolyethylene glycol to the oxidation product is (4-6): 1, to obtain a PEG-modified polypeptide;
wherein X is threonine or serine.

9. A polypeptide analog prepared by the method according to any one of claims 1 to 8.

10. A GLP-1 receptor agonist analog prepared by the method according to any one of claims 1 to 8.

11. The GLP-1 receptor agonist analog according to claim 10, wherein the GLP-1 receptor agonist analog has a structure of PEG-X-GLP-1 receptor agonist;
wherein X is threonine or serine.

12. The GLP-1 receptor agonist analog according to claim 10, wherein the GLP-1 receptor agonist comprises any one of GLP-1, exenatide, liraglutide, albiglutide, dulaglutide, lixisenatide, benaglutide or semaglutide;
preferably, the PEG is methoxypolyethylene glycol;
preferably, an end group of methoxypolyethylene glycol comprises any one of an amino group, an oxyamino group or hydrazide;
preferably, the molecular weight of methoxypolyethylene glycol is 2000 Da to 50000 Da, preferably 5000 Da to 20000 Da, further preferably 5000 Da to 10000 Da.

13. A pharmaceutical composition, comprising the polypeptide analog according to claim 9 and/or the GLP-1 receptor agonist analog according to any one of claims 10 to 12.

14. The pharmaceutical composition according to claim 13, further comprising any one or a combination of at least two of a pharmaceutically acceptable carrier, excipient or diluent.

15. Use of any one or a combination of at least two of the polypeptide analog according to claim 9, the GLP-1 receptor agonist analog according to any one of claims 10 to 12 or the pharmaceutical composition according to claim 13 or 14 in the preparation of a medicament for the prevention and/or treatment of obesity, diabetes or Alzheimer's disease.
